# EUROPÄISCHE PATENTANMELDUNG

(11) **EP 1 958 618 A1**
(43) Veröffentlichungstag der Anmeldung: **20.08.2008**
(21) Anmeldenummer: 07102446.7
(22) Anmeldetag: 15.02.2007
(51) Int. Cl.: A61K 9/19, A61K 9/00, F26B 5/06, A61K 47/02, A61K 47/12

(54) **Verfahren zur Gefriertrocknung mit optimierter Rekonstitution von Biopolymeren**

(71) Anmelder: Octapharma AG, 8853 Lachen (CH)
(72) Erfinder: Björnstrup, Kim, CH-8835 Feusisberg (CH); Kern, Martin, AT-2525 Schönau (AT); Heger, Andrea, AT-1110 Wien (AT); Gruber, Gerhard, AT-1210 Wien (AT); Sachse, Hans, AT-1040 Wien (AT); Schütz, Raimund, AT-1220 Wien (AT); Römisch, Jürgen, AT-1440 Gramatneusiedl (AT); Svae, Tor-Einar, AT-2340 Mödling (AT)
(74) Vertreter: Meyers, Hans-Wilhelm

(57) **Zusammenfassung**

Verfahren zur Rekonstitution eines Lyophilisats enthaltend Inhaltsstoffe, wobei
- die Rekonstitution eine Behandlung des Lyophilisats mit einer im wesentlichen wässrigen Lösung umfasst, die Substanzen enthält, die eine Einstellung eines vorbestimmten pH-Wert-Bereiches einer aus der Rekonstitution entstehenden Lösung bewirken, oder
- mit einer im wesentlichen wässrigen Lösung, wenn die Inhaltsstoffe bei dem Gefriertrocknungsprozess in einer Lösung vorlagen, die Substanzen enthielt, die die Einstellung eines vorbestimmten pH-Wert-Bereiches in der bei der Rekonstitution entstehenden Lösung bewirkten.

## Beschreibung

Gegenstand der vorliegenden Erfindung ist ein Verfahren zur Rekonstitution eines Lyophilisats enthaltend Inhaltsstoffe sowie die Verwendung eines Behälters in dem erfindungsgemäßen Verfahren.

Die Lagerung von Biopolymeren, wie Proteinen, Polysacchariden oder Nukleinsäuren und deren Mischungen erfolgt je nach Anforderung an das Produkt in löslichem, gefrorenem (wässrigen) oder lyophilisiertem Zustand. Im Falle von Medikamenten werden alle Lagerungs- bzw. Darreichungsformen entsprechend Stabilität oder Applikationsart verwendet. Dies gilt zum Beispiel auch für Lösungen, die einzelne (gereinigte) Proteine enthalten oder Mischungen von Proteinen (Biopharmazeutika), oder deren Kombination mit anderen Substanzen, wie Lipiden. Optional werden für die jeweiligen Anforderungen speziell optimierte Stabilisatoren hinzugegeben, um das Produkt möglichst lange in einem nativen und aktiven Zustand zu halten.

Beispielsweise werden aus Blut-Plasma isolierte Proteine, wie Gerinnungsfaktoren (Faktor II, V, VII, VIII, von Willebrand Faktor (VWF), VWF/FVIII-Komplex, FIX, X, XI, XII, XIII, Fibrinogen, deren Kombinationen sowie deren aktivierte Formen), FVII-aktivierende Protease (FSAP), Protease-Inhibitoren, z. B. α -1 - Antitrypsin, Antithrombin III, Heparin-Cofaktor II, C1-Esterase Inhibitor, Antiplasmin, Protein C und dessen Cofaktor Protein S, Protein Z und andere Inhibitoren, Immunglobuline wie IgG, IgA, IgE, IgM und entsprechende Subklassen, Proteine der Fibrinolyse und des Komplementsystemes wie Plasminogen, Plasminogen-Aktivatoren, Komplementfaktoren, Transportproteine wie Albumin, Transferrin, Wachstumsfaktoren und die Wundheilung unterstützende Proteine, wie Hepatocyte Growth Factor, HGF, Platelet-Derived Growth Factor, PDGF, Fibroblast Growth Factor, FGF, Fibronectin, Vitronectln etc. und deren Kombinationen, sowie Plasma selbst und dessen Subfraktionen, wie Cryo-Präzipitat, und andere dem Fachmann vertrauten Subfraktionen, wie Intermediate aus der Plasmafraktionierung nach Cohn, Kistler-Nitschmann und deren Modifikationen, in unterschiedlichen Zuständen gelagert.

Immunglobuline werden je nach Produkt in flüssiger oder lyophilisierter Form verwendet, wobei hinsichtlich der Anwendung und Zweckmäßigkeit die flüssige Variante bevorzugt wird, da sie das vorherige Rekonstituieren unnötig macht.

Im Falle des Plasmas selbst, das z.B. für Transfusionszwecke Verwendung findet, sind sowohl flüssig gelagerte, flüssig-gefrorene als auch lyophilisierte Varianten bekannt und werden je nach Anforderung genutzt.

Mit Intermediaten aus Herstellprozessen rekombinanter und transgener Produkte sowie der Endprodukte kann entsprechend verfahren werden. Vorteile eines lyophilisierten Produktes sind die mögliche vergleichsweise längere Lagerung bei Temperaturen über 0°C, bis zu Raumtemperatur und darüber hinaus, kombiniert mit in der Regel einem (durch den verringerten Wasseranteil) reduzierten Gewicht. Zwar setzt ein lyophilisiertes Produkt die Rekonstitution in geeigneten Lösungen voraus, jedoch überwiegen die Vorteile in bestimmten Situationen. Besonders in der Notfallmedizin, bevorzugt bei Einsätzen unter sehr schwierigen Behandlungsbedingungen, beispielsweise außerhalb eines professionellen Hospitalbereiches, ist eine Glasflasche durch Gewicht und möglicher Bruchgefahr einem flexibleren und leichterem Material unterlegen. Zudem ist die Kühlung oder das Auftauen des mitzuführenden gefrorenen Produktes (wie Plasma) zeitaufwändig.

Die Lyophilisierung von Plasma oder anderen Proteinlösungen in Glasflaschen oder Ampullen ist dem Fachmann bekannt. Nach Beendigung des Gefriertrocknungsprozesses werden die Gefäße mit einem geeigneten Stopfen verschlossen, um das Wiedereintreten von Wasserdampf zu minimieren. Dies ist in der Regel unproblematisch, da die Gefäßöffnungen relativ zum gesamten Gefäß und zur Lyophilisatoberfläche sehr klein/eng sind und sich somit die Wiederaufnahme von Wasser(dampf) bei normaler Umgebungsfeuchte relativ langsam vollzieht. Das Auflösen des Produktes im Bedarfsfall wird durch Zugabe von Wasser oder anderen geeigneten Lösungen erreicht.

In bestimmten Situationen unvorteilhafte Eigenschaften der Gefriertrocknung und Lagerung in Glasbehältern wurden bereits zum Teil oben erwähnt. Hinzu kommt, dass, abhängig von der Gefäßöffnung, eine für die Gefriertrocknung relativ geringe Austrittsfläche zur Verfügung steht und sich damit eine entsprechend lange Lyophilisierungsdauer ergibt. Wünschenswert ist daher auch ein Verfahren, das es ermöglicht, die Lyophilisierung zu beschleunigen, ohne das es in nennenswertem Umfang zur schnellen Aufnahme von Feuchtigkeit beim späteren Verschließungsvorgang kommt.

Der Lyophilisationsvorgang entfernt Wasserdampf aus dem gefrorenen Material unter dem Fachmann bekannten Ausführungsformen. In Lösungen, die zum Beispiel Kohlendioxid enthalten, kann dieses (zumindest partiell) ebenfalls entfernt werden, wodurch sich eine Verschiebung des pH-Wertes in Richtung Basizität des resultierenden Produktes nach Rekonstitution mit Wasser oder anderen Lösungen ergeben kann. Dies wird beispielsweise bei proteinhaltigen Lösungen, wie Blutplasma, beobachtet. Ein pH-Wert von 7,0-7,6 vor Gefriertrocknung, kann nach Rekonstitution einen pH-Wert von über 8,0 erreichen, wie in dem unten angeführten Beispiel näher erläutert wird. Diese pH-Wert Verschiebung kann Konsequenzen für Produkt und Patient haben. Im Besonderen die Integrität bestimmter Wirkkomponenten bzw. deren Stabilität über einen längeren Zeitraum kann beeinträchtigt sein. Zudem bedarf der basischere pH-Wert des rekonstituierten Produktes der gesteigerten Aufmerksamkeit des transfundierten Patienten bei Applikation, wenn es sich um größere Volumina handelt.

Ein der Erfindung zu Grunde liegendes technisches Problem bestand in der Bereitstellung eines verbesserten Verfahrens, mit dem die beschriebenen Nachteile in der Handhabung von Lyophilisaten, insbesondere in der medizinischen Anwendung, vermieden oder zumindest abgeschwächtwerden.

Abgeholfen wird diesem Problem durch ein Verfahren zur Rekonstitution eines Lyophilisats enthaltend Inhaltsstoffe, wobei
- die Rekonstitution eine Behandlung des Lyophilisats mit einer im wesentlichen wässrigen Lösung umfasst, die Substanzen enthält, die eine Einstellung eines vorbestimmten pH-Wert-Bereiches einer aus der Rekonstitution entstehenden Lösung bewirken, oder
- mit einer im wesentlichen wässrigen Lösung, wenn die Inhaltsstoffe bei dem Gefriertrocknungsprozess in einer Lösung vorlagen, die Substanzen enthielt, die die Einstellung eines vorbestimmten pH-Wert-Bereiches in der bei der Rekonstitution entstehenden Lösung bewirkten.

Das erfindungsgemäße Verfahren ermöglicht es dem Fachmann eine bei der Rekonstituierung zu erwartende oder sich einstellende pH-Wert Verschiebung durch geeignete Maßnahmen auszugleichen.

Es kann im erfindungsgemäßen Verfahren vorgesehen sein, dass die Substanzen eine Einstellung des pH-Wertes in Bereiche verhindern, die die Struktur der Inhaltsstoffe wie Proteine im Lyophilisat durch zumindest teilweisen Verlust an Aktivität und/oder Nativität, Denaturierung, Fragmentierung, Aggregation, chemische Modifikation wie Oxidation negativ beeinflussen.

Beispielsweise kann durch Senkung des pH-Wertes in der zu lyophilisierenden Lösung in einen Bereich, der die Inhaltsstoffe, z. B. Hauptwirkkomponenten des Produktes, nicht beeinträchtigt, erreicht werden, dass sich der pH-Wert des Produktes nach Lyophilisation und Rekonstitution in dem vorbestimmen pH-Wertbereich befindet. Für Plasma kann dieser vorbestimmte Bereich typischerweise ein pH-Wertbereich von pH 6,95 bis 7,9, insbesondere pH 7.0 bis 7.6 sein, kann jedoch für andere Biopolymerlösungen sehr unterschiedliche pH-Werte erfordern. Die jeweils einzustellenden pH-Werte können, soweit sie dem Fachmann ohnehin bekannt sind, in einfachen Versuchen ermittelt werden, in dem z. B. unter verschiedenen pH-Wertbedingungen wesentliche Parameter der Inhaltsstoffe, wie Nativität, Wirksamkeit, Aggregationsverhalten usw., bestimmt werden.

In einer Ausführungsform des erfindungsgemäßen Verfahrens ist die Einstellung des pH-Wertes die Senkung des pH-Wertes im Sinne einer resultierenden saureren Lösung vor Lyophilisierung, durch Zugabe geeigneter Substanzen, Pufferlösungen oder (verdünnter) Säurelösungen, um nach Rekonstitution des Produktes einen gewünschten pH-Wert zu erreichen.

Die Einstellung des pH-Wert Bereiches der Lösung vor der Lyophilisierung kann so erfolgen, dass sich ein pH-Wert der nach der Rekonstituierung in der daraus entstehenden Lösung vorliegen soll (vorbestimmter pH-Wert oder pH-Wertbereich) einstellt, wenn mit einer Rekonstituierungslösung das Lyophilisat rekonstituiert wird. Ohne Einstellung kann der pH-Wert der rekonstituierten Lösung nämlich von dem pH-Wert der Lösung vor der Gefriertrocknung deutlich abweichen, wenn dies tolerabel ist . So kann beispielsweise eine Lösung mit Proteinen als Inhaltsstoffen mit einem pH-Wert von 7,4 auf 6,0 eingestellt werden, um nach der Rekonstituierung z. B. mit Wasser einen pH-Wertbereich von 6,6 bis 7,0 zu erreichen.

Eine weitere Möglichkeit, den angestrebten pH-Wert nach Rekonstitution des Produktes zu erreichen, ist die Einstellung des pH-Wertes während oder nach Lösen des Lyophilisats. Dies kann durch Einstellen der Produktlösung mit geeigneten Pufferlösungen oder durch nachträgliches Einbringen entsprechend angesäuerter Lösungen erfolgen. Eine individuelle Titration jedes einzelnen Gefäßes oder entnommener Lösungen erfordert Zeit und ist im Sinne einer raschen und sicheren Applikation, wie z.B. einer Transfusion, wenig praktikabel. Jedoch ist das Lösen des Produktes mit einer geeigneten Lösung, die an sich saure Eigenschaften aufweist und zu dem angestrebten pH-Wert des Produktes führt, eine ebenfalls praktikable Maßnahme.

Im erfindungsgemäßen Verfahren werden beispielsweise als Substanzen Puffersubstanzen wie Salze aus mineralischen Säuren und Basen eingesetzt, insbesondere Phosphate oder Carbonate, die auch durch Einbringen von CO₂ entstehen können. Weiterhin können Di- oder Monohydrogenphosphate, Hydrogencarbonate, Hydrogensulfate, Salze organischer Säuren oder Basen, Tris-Hydroxymethylaminomethan und/oder Aminosäuren als Substanzen zur Einstellung des vorbestimmten pH-Wertes eingesetzt werden. Insbesondere kann die Rekonstitution des Lyophilisats in einer wässrigen Lösung erfolgen, die physiologisch verträgliche sauer hydrolysierende Salze oder physiologisch verträgliche Säuren enthält.

Die Rekonstitution kann vorteilhafterweise mit im wesentlichen neutralem Wasser für Injektionszwecke erfolgen, wenn die Gefriertrocknung der Inhaltsstoffe in einer Lösung erfolgt, die Substanzen enthält, die die Einstellung eines vorbestimmten pH-Wert-Bereiches in der bei der Rekonstitution entstehenden Lösung bewirken.

Die erfindungsgemäße Vorgehensweise wird beispielhaft näher erläutert. Die Ansäuerung des Produktes vor Lyophiliserung kann mit geeigneten Lösungen und Substanzen erfolgen, die eine entsprechende Einstellung auf den pH-Wertbereich nach Lösen des erhaltenen Produktes ermöglicht. Zitronensäure in geeigneter Konzentration kann beispielsweise verwendet werden, um den pH-Wert vom Plasma in den pH-Wert Bereich von pH 6,0-6,95 zu bringen, bevorzugt pH 6,5-6,95, um nach Rekonstitution des lyophilisierten Plasmas einen pH-Wert von 7,0 bis 7,9 zu erreichen. Besonders bevorzugt ist hier die Zugabe von Zitronensäure im Konzentrationsbereich von 0,1-20,0 mM (Endkonzentration der zugegebenen Lösung im Produkt), bevorzugt 1-5 mM für Plasma. In diesen Konzentrationsbereichen bewegen sich ebenfalls Zitronensäure-Lösungen zur Rekonstitution von lyophilisiertem Plasma. Für andere Proteinlösungen im Sinne der pH-Wert Einstellung vor Lyophilisierung und bei Rekonstitution bietet sich die Zugabe von Zitronensäure im Konzentrationsbereich 0,1-50 mM (Endkonzentration im Produkt der zugegebenen Säure) an. Es sei darauf hingewiesen, dass die resultierende Zitronensäure bzw. resultierender Zitrat-Gehalt im Produkt insgesamt höher sein kann, wenn bereits vor Zugabe Zltronensäure/Zitrat anwesend war. Dies gilt in entsprechender Weise auch für andere Substanzen, die für eine solche Einstellung hinzugegeben werden können, wie Phosphatpuffer etc.

Entsprechend sind andere geeignete Mittel und die Zugabe anderer Puffer- und Säurelösungen ebenfalls möglich. Dabei ist zu erwähnen, dass der pH-Wert der Lösung auch durch Einbringen von CO₂ Gas in die Lösung (Begasung der Oberfläche unter Rühren der Lösung oder durch Einbringen eines Schlauches in die Lösung oder ähnliche dem Fachmann vertraute Techniken) vor dem Lyophilisieren erfolgen kann.

Dagegen können lyophilisierte Gefäße auch vor Verpackung, wie oben beschrieben, einer sauren Atmosphäre ausgesetzt werden. Beispielsweise kann durch Einbringen von CO₂ in Form von Gas eine Diffusion in die Behälter erfolgen, so dass das resultierende Lyophilisat und später die entsprechende Produktlösung den gewünschten pH-Wert erreicht.

Die beschriebenen Maßnahmen zur Einstellung des gewünschten pH-Wertes vor Lyophilisierung, während der Produktlagerung sowie der Rekonstitution können durch die beschriebenen Maßnahmen erreicht werden. Diese können einzeln oder in Kombination Einsatz finden. Es ist somit aber auch möglich, das Lyophilisat unter sauren pH-Wert Bedingungen zu lagern, wie beispielsweise vorteilhaft und bekannt für Immunglobuline, um diese dann in Wasser zu lösen und das saure Milieu beizubehalten und eine rasche Aggregation des oder der Inhaltsstoffe zu verhindern.

Eine Einstellung des pH-Wertes für Produktlösungen vor Patientenapplikation kann auch notwendig sein, um den erforderlichen Behandlungseffekt zu erzielen.

In der zu lyophilisierenden Lösung und/oder einer Rekonstitutionslösung können Stabilisatoren der Inhaltsstoffe, insbesondere von therapeutisch wichtigen Inhaltsstoffen vorhanden sein. Als Stabilisatoren sind insbesondere, Polysaccharide, Oligosaccharide, z. B. Zucker, Polyole, z.B. Mannitol oder Sorbitol, Aminosäuren oder Kombinationen davon zu nennen.

In einer weiteren Ausführungsform des erfindungsgemäßen Verfahrens wird das Lyphilisat dadurch erhalten, dass die Inhaltsstoffe in einer im wesentlichen wässrigen Lösung vorliegen und in einen Behälter gefüllt werden, der zumindest teilweise aus für Wasser undurchlässigem aber für Wasserdampf durchlässigem und sterilem Material besteht und nach Beendigung des Gefriertrocknungsprozesses eine Aufnahme von Wasserdampf über einen vorher bestimmten Restfeuchtegehalt des Lyophilisats hinaus verhindert wird.

In diesem Zusammenhang soll nicht unerwähnt bleiben, dass der sogenannte Restfeuchteanteil des Lyophilisates in vielen Fällen sehr wichtig für die Haltbarkeit des Produktes ist. Lyophilisate mit empfindlichen Inhaltsstoffen z. B. therapeutische Proteine sollten je nach Lagerungsbedingungen (Temperaturen) unter 5% Restfeuchte gelagert werden, bevorzugt unter 3%, um lang anhaltende Aktivität, Nativität und Sicherheit zu ermöglichen.

Die rasche Wiederaufnahme von Wasserdampf in das Kunststoff-Gefäß kann im erfindungsgemäßen Verfahren durch mehrere Maßnahmen verhindert oder erschwert werden. Die Reduktion der relativen Luftfeuchte im Entnahmebereich und/oder das Fluten des Lyophilisators mit Stickstoff oder andere geeignete Maßnahmen sind praktikabel. Insbesondere kann die rasche Abdeckung bzw. Versiegelung der Membranfläche durch geeignete Maßnahmen, wie Aufbringen von wasserdampf-undurchlässigen und sterilen Folien oder das rasche Verschließen der Gefäße in geeignete Folienbeutel die Feuchtigkeitsaufnahme erschweren oder gar unterbinden. Dies kann unter Stickstoff oder Evakuierung der Beutel erfolgen. Zudem oder alternativ kann dem Beutel dem Fachmann bekanntes Material zur Bindung von Wasser(dampf) beigegeben werden, je nach dem welcher Restfeuchtebereiche eingestellt werden soll.

Es kann sich empfehlen, den Behälter aus für Wasser undurchlässigem aber für Wasserdampf durchlässigem und sterilem Material auf einem Stützmaterial anzuordnen.

Es wurden bereits verschiedentlich Inhaltsstoffe erwähnt, die im Lyophilisat, das gemäß der Erfindung rekonstituiert werden kann vorhanden sein können. Das erfindungsgemäße Verfahren ist aber nicht auf diese Inhaltsstoffe beschränkt.

Die mittels des erfindungsgemäßen Verfahrens rekonstituierbaren Lyophilisate können vielmehr Inhaltsstoffe enthalten, die ausgewählt sind aus der Gruppe bestehend aus Biopolymeren wie Proteinen, Polysacchariden, Nukleinsäuren, Lipiden und deren Mischungen. Therapeutisch bedeutsam sind als Inhaltsstoffe insbesondere Proteine aus Blut, Plasma oder Serum, sowie rekombinant oder transgen hergestellte Proteine.

Beispielhaft zu nennen sind insbesondere aus Blut-Plasma isolierte Proteine, wie Gerinnungsfaktoren (Faktor II, V, VII, VIII, von Willebrand Faktor (VWF), VWF/FVIII-Komplex, FIX, X, XI, XII, XIII, Fibrinogen, deren Kombinationen sowie deren aktivierte Formen), FVII-aktivierende Protease (FSAP), Protease-Inhibitoren, z. B. α -1 -Antitrypsin, Antithrombin III, Heparin-Cofaktor II, C1-Esterase Inhibitor, Antiplasmin, Protein C und dessen Cofaktor Protein S, Protein Z und andere Inhibitoren, Immunglobuline wie IgG, IgA, IgE, IgM und entsprechende Subklassen, Proteine der Fibrinolyse und des Komplementsystemes wie Plasminogen, Plasminogen-Aktivatoren Komplementfaktoren, Transportproteine wie Albumin, Transferrin, Wachstumsfaktoren und die Wundheilung unterstützende Proteine, wie Hepatocyte Growth Factor, HGF, Platelet-Derived Growth Factor, PDGF, Fibroblast Growth Factor, FGF, Fibronectin, Vitronectln etc. und deren Kombinationen, sowie Plasma (in Form von z. B. Zitrat-Plasme, Oxalat- oder heparin-Plasma) selbst und dessen Subfraktionen, wie Cryo-Präzipitat, und andere dem Fachmann vertrauten Subfraktionen, wie Intermediate aus der Plasmafraktionierung nach Cohn, Kistler-Nitschmann und deren Modifikationen.

Gleichermaßen kommen aber auch Inhaltsstoffe, die in biologischen Quellen wie Körperflüssigkeiten aus transgenen oder Fraktionen mit rekombinant hergestellten Proteinen enthalten sind, in Betracht.

In einer besonderen Ausführungsform wird das erfindungsgemäße Verfahren in Behältern ausgeführt, bei denen das für Wasserdampf durchlässige sterile Material aus fluorierten Kunststoffmaterialien, Polyester oder Polyethylen besteht.

Um die als nachteilig empfundene relativ lange Dauer der Lyophilisation abzukürzen, wird in dieser Ausführungsform des erfindungsgemäßen Verfahrens eine möglichst große Oberfläche, geeignet für den Wasserdampfaustritt, gegenüber der (gefrorenen) Produktoberfläche, zur Verfügung stellt. Zur Reduzierung des gesamten Behältergewichtes und der leichteren Handhabung können auch als Stützmaterial für die Behälter in denen die Lyophilisierung erfolgt, Kunststoffmaterialien in Form von geeigneten Gefäßen, wie Beuteln, verwendet werden. Diese können aus Materialien, wie Polypropylen, Polyethylen, Ethylvinylacetat, Polyvinylchlorid, Polyurethan oder anderen geeigneten Materialien oder deren Kombinationen und Schichten bestehen, wie vom Typ der Kraton^{®} Polymere, die aus dem Fachmann bekannten Mehrschichtsystemen bestehen können und deren Kombinationen mit anderen Materialien.

Diese Materialien sind in der Regel nicht durchlässig für Wasserdampf, so dass in Form einer wasserdampf-durchlässigen Membran dafür gesorgt werden kann, dass ein geeignet hoher Gasaustausch bei Gefriertrocknung entstehen kann. Solche Membranen können aus Polytetrafluoroethylen (Fluoropolymere) Polypropylen, Polyethylen oder anderen geeigneten Membranen und deren Kombinationen bestehen. Nicht jedes Membranmaterial ist mit dem Beutelmaterial einfach kompatibel im Sinne einer dichten herzustellenden Verbindung z.B. Verschweißung. Entsprechende Kombinationen werden durch dem Fachmann vertrauten Techniken miteinander vereint, beispielsweise durch thermische Verschweißung und/oder mechanische Verankerung oder andere geeignete Verfahren. Bevorzugte Kombinationen sind Gefäßmaterlallen aus Polypropylen und/oder Kraton Polymere mit Fluoropolymer-Material basierten Membranen, bevorzugt Polytetrafluoroethylen oder Polypropylene, sowie bevorzugt Gefäße aus Polyethylen mit Polyethylen-basierten Membranen, wie den sogenannten High Density Polyethylenen. Mehrschichtige Systeme bieten sich ebenfalls als Basis für Gefäße an, um Stabilität, Sterilität und Wasser- und Gas-Dichtigkeit zu gewährleisten, wobei diese mit geeigneten Membranmaterialien verbunden werden können.

Die Membranen können den gesamten Beutel bilden, jedoch wird in der Regel bevorzugt sein, nur einen Teil des Beutels in Form einer solchen Membran zu erhalten. Ein Grund dafür ist, dass die Lyophilisierung in der Regel so erfolgt, dass das Plastikgefäß in dem Lyophilisator auf eine (gekühlte, zu kühlende) solide Fläche aufliegt und somit ein Teil der Membranfläche nicht für den maximalen Wasserdampfaustritt zur Verfügung steht. Des Weiteren wird der Wiedereintritt von Wasserdampf in das Lyophilisat nach dem Öffnen des Lyophilisators in der Regel deutlich beschleunigt sein gegenüber einem Plastikgefäß mit einem optimierten Membrananteil.

Eine besondere Ausführungsform eines solchen Gefäßes stellt beispielsweise ein Beutel aus Polypropylen dar, dessen Oberfläche einseitig aus einem großen Anteil (>50% dieser einseitigen Fläche, die dem Lyophilisatorraum zugewandt ist) der Membran besteht, der bei der Lyophilisierung frei zugänglich ist, d.h. abgewandt von der Kühlfläche, auf die das Gefäß aufgelegt wird. Der Membrananteil dieser zugänglichen Fläche sollte möglichst groß sein, damit der Gefriertrocknungsvorgang effektiv erfolgen kann. Wird beispielsweise die Membranfläche gegenüber der Beutelfläche zu klein sein (wenn z.B. die Membrankante zu weit vom Beutelrand und der darunter befindlichen Lösung ist), so müssen sich die Wasserdampfmoleküle bei der Verdampfung zunächst horizontal durch Lösung/Lyophilisat bewegen, um dann durch die Membran austreten zu können. Dies kann, neben einer verlängerten Lyophilisierungsdauer, zu unerwünschten Inhomogenitäten des Lyophilisates (Bereiche mit unvollständiger Trocknung, Einschlüsse) führen.

Im Sinne des erfindungsgemäßen Verfahrens ist auch die Verbringung des für Wasserdampf durchlässigen Behälters in einen für Wasserdampf undurchlässigen Behälter, der aus Kunststoffen oder Aluminiumfolie bestehen kann.

Das erfindungsgemäße Verfahren kann in den dem Fachmann geläufigen Lyophilisierungsverfahren durchgeführt werden. Insbesondere kann dies auch in einem Verfahren erfolgen, bei dem ein Gefriertrocknungsprozess angewendet wird, der einen ersten und einen zweiten Schritt umfasst, wobei im zweiten Schritt des Gefriertrocknungsprozess eine geringere Desorptionsenergieeintragung in den Gefriertrocknungsprozess als im ersten Schritt erfolgt. Dieses Verfahren ist Gegenstand der europäischen Patentanmeldung EP 06115736.8 und dort näher beschrieben. Auf die EP 06115736.8 wird hier ausdrücklich bezug genommen.

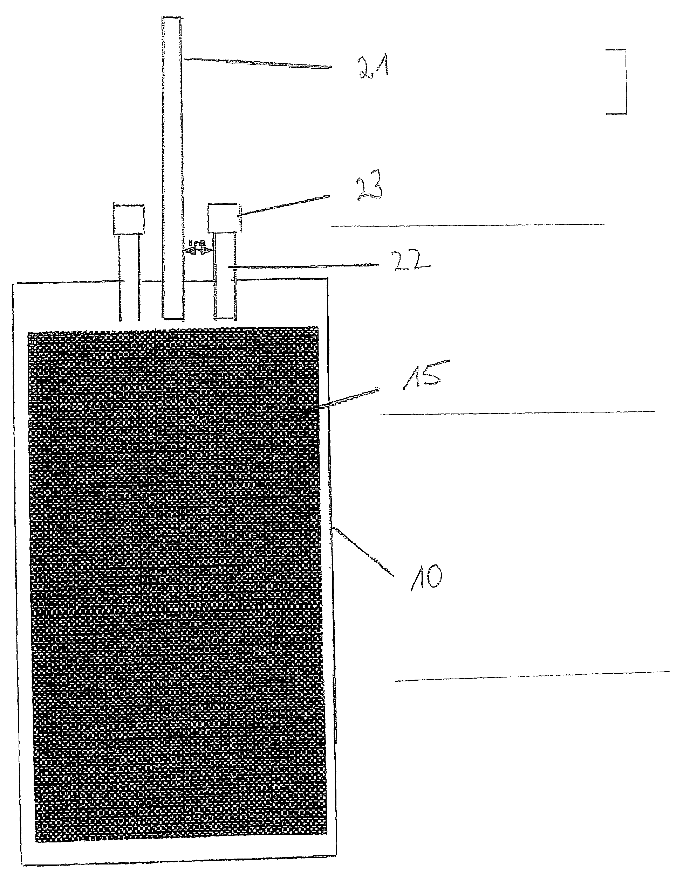

Das erfindungsgemäße Verfahren lässt sich insbesondere unter Verwendung eines Behälters wie er in der Figur schematisch dargestellt ist ausführen. Der Behälter zur Lyophilisierung weist Wände 10 sowie Anschlusselemente 21, 22 auf. Die Wände 10 bestehen zumindest teilweise aus für Wasser undurchlässigem aber für Wasserdampf durchlässigem und sterilem Material 15. Das Anschlusselement 22 kann als Anschluss-Stutzen ausgebildet und durch ein Septum 23 abgeschlossen sein. Die Befüllung des Beutels kann beispielsweise durch den Füllschlauch 21 erfolgen. Die Wand 10 wird hier im sichtbaren Teil durch das Fenster 15 unterbrochen. Das Fenster 15 besteht aus einem für Wasserdampf durchlässigem aber für Wasser undurchlässigem Material. Dieses Material ist weiter oben im Detail beschrieben.

Die Erfindung wird im Folgenden an Hand der Beispiele näher erläutert.

### Beispiel 1

Ziel dieses Versuches war es, prinzipiell die Kinetik der Wiederaufnahme von Wasserdampf durch das lyophilisierte Produkt und den Einfluss der Umgebungstemperatur und der Luftfeuchtigkeit zu untersuchen. Dazu wurden Glasflaschen (20 ml) mit schmalem Hals/Einlass mit je 5 ml Plasma befüllt (1 cm Füllhöhe) und lyophilisiert. Die Kontrollansätze wurden luftdicht verschlossen, wogegen andere Flaschen entweder bei Raumtemperatur/60% Luftfeuchte oder bei 40°C/75% Luftfeuchte bis zu 7 Stunden gelagert wurden, jeweils der Flaschen-Gasraum mit Stickstoff kurz begast und die Flaschen luftdicht verschlossen wurden. Die Analyse der Restfeuchtegehalte der Lyophilisate wurde nach der dem Fachmann bekannten Karl-Fischer-Methode durchgeführt.

### Ergebnis:

| Raumtemp. 60 % | Restfeuchtegehalt [%] | Zunahme der Restfeuchte [%] |
|---|---|---|
| 0 h | 0,86(0,81-1,01) | - |
| 1 h | 3,30 (2,82-3,54) | + 2,44 % |
| 2 h | 5,81 (5,65-5,95) | + 4,95 % |
| 7 h | 10,91 (10,57-11,20) | + 10,05% |

| 40 °C 75 % Luftfeuchtigkeit | Restfeuchtegehalt [%] | Zunahme der Restfeuchte [%] |
|---|---|---|
| 0 h | 0,86(0,81-1,01) | - |
| 1 h | 5,95(5,93-6,01) | + 5,09 % |
| 2 h | 8,97(8,57-9,21) | + 8,11 % |
| 7 h | 16,65(15,93-17,07) | + 15,79% |

Die Ergebnisse zeigen, dass die Restfeuchtezunahme des Lyophilisates relativ schnell erfolgt und von der Umgebungstemperatur und Luftfeuchtigkeit abhängt. Entsprechend können Maßnahmen getroffen werden, um einen unerwünscht hohen Restfeuchtegehalt zu limitieren. Die Kontrollproben (0 Stunden, also direkt nach Lyophilisatorentnahme) zeigen, dass die Restfeuchte bei geeignetem Verfahren (rascher luftdichter Verschluss) in einem gewünschten Bereich gehalten werden können.

### Beispiel 2

In diesem Beispiel wurde den oben gewonnenen Erkenntnissen Rechnung getragen, indem die lyophilisathaltigen Gefäße nach Entnahme aus dem Lyophilisator zeitnah luftdicht in einem Plastikbeutel verschlossen wurden. Dazu wurden Polypropylen-Gefäße in Form eines Beutels verwendet wie in der Figur skizziert, wobei die Polytetrafluorethylen-Membran einseitig flächig aufgebracht wurde (nach vorheriger Anfertigung eines entsprechenden Fensters auf einer Beutelseite) und so eine für Flüssigkeit dichte Verbindung resultierte. Ein- und Auslasselemente für das Befüllen des Beutels, gegebenenfalls rasche Entlüftung im Sinne einer Verdrängung der im Beutel befindlichen Luft durch die eintretende Flüssigkeit sind vorhanden. Darüber hinaus ermöglichen die Ein- und Auslasselemente auch die Eintragung einer Rekonstitutionsflüssigkeit nach Gefriertrocknung und Auslass für die zu entnehmende rekonstituierte Lösung, in der praktischen Anwendung für die Infusion am Patienten. Diese Ein-/Auslassverbindungen können aus dem Fachmann bekannten Plastikmaterialien bestehen, in dem beschriebenen Beispiel aus Polypropylen.

Nach Sterilisierung der Gefäße wurden in diese Beutel jeweils 200 ml Blutplasma unter sterilen Bedingungen eingefüllt, die Einlassverbindung verschweisst, die Beutel eingefroren und der Gefriertrocknung zugeführt. Nach Trocknung wurden die Beutel dem Lyophilisator entnommen und umgehend in Plastikbeutel eingeschweißt und luftdicht verschlossen.

Für die Rekonstitution der Lyophilisate wurden die lyophilisathaltigen Gefäße den Schutzbeuteln (nach vorherigem Öffnen durch Aufschneiden) entnommen und anschließend mit 184 ml Flüssigkeit, wie unten beschrieben, versetzt, um ein Endvolumen von 200 ml rekonstituiertes Plasma zu erhalten. Unter leichtem Schwenken der Beutel erfolgte die vollständige Rekonstitution zu einer klaren Lösung innerhalb von 5 Minuten.

### Restfeuchtegehalt:

Die Analyse der Lyophilisate nach Lagerung der Beutel zeigte Restfeuchtegehalte zwischen 1,0 und 1,5% der sehr schnell in die Beutel eingeschlossenen Gefäße und 2,0 bis 2,5% Restfeuchte, wenn die Gefäße mit Verzögerung verpackt wurden. Diese Befunde waren reproduzierbar für Gefäße, die über Tage und mehrere Wochen in Beuteln verschlossen gelagert worden waren. Dagegen zeigten Lyophilisate, die für Stunden nicht in entsprechende Beutel eingeschweißt worden waren, Restfeuchtegehalte von deutlich mehr als 3%, wie aus dem Ergebnis aus Beispiel 1 prinzipiell vermutet werden konnte.

### Beispiel 3

Es wurde verfahren wie in Beispiel 2 ausgeführt, inklusive Nutzung der Plastikmaterialien, Gefriertrocknungsbedingungen und Handhabung. Die Gefäße in Form von Membrananteil-enthaltenden Beuteln wurden mit 200 ml Plasma befüllt. In diesem Beispiel wurde ein gerinnungsaktives solvent/detergent behandeltes humanes Blutplasma und das universell verwendbare Blutplasma gemäß WO2005/058334 bzw. EP-A-0991416 verwendet. Beide gerinnungsaktiven Produkte werden nach Auftauen und Vereinen von bis zu 1.520 Plasma-Einzelspenden mittels eines streng kontrollierten Herstellungsverfahrens unter GMP-Bedingungen produziert. Durch das in den Produktionsprozess implementierte Solvent/Detergent Verfahren wird ein sehr hohes Mass an Sicherheit im Sinne einer Minimierung eines infektiösen Restrisikos (für transfusionsrelevante umhüllte Viren) erzielt, Einzelspender-Screening und weitere Sicherheits-Maßnahmen und effektive Prozessschritte tragen auch maßgeblich zur Sicherheit der Produkte bei. Universell verwendbares Plasma wird durch die optimale Integration von Einzelspenden der Blutgruppen A, B und AB in beschriebenen Mischungsverhältnissen erzielt (wie in EP -A- 0 991 416 und EP -A- 1 696 940 beschrieben), das die Blutgruppenunabhängige Anwendung am Patienten ermöglicht.

Der sich anschließende Herstellungsprozess für ein humanes Blutplasma erfolgte nach P. Hellstern et al., Vox Sang 1992; 63: 178-185.

Auch Einzelspenderplasmen oder durch andere Verfahren behandelte Einzel- und Plasma-Pool-Produkte, können Verwendung finden, sowie Polymere im Sinne der Erfindung. Drei verschiedene Ansätze wurden in diesem Beispiel untersucht, unter Mitführung eines Kontrollansatzes:
A. Dem S/D behandelten Plasma-Pool (humanes Blutplasma und universell verwendbares Plasma-Verfahren) wurde Zitronensäure durch Eintropfen, bei guter Durchmischung durch Rühren, auf eine Endkonzentration der 'zugegebenen Zitronensäure' von 3-4 mM zugegeben, wobei der pH-Wert von pH 7,0-7,4 auf pH 6,5-6,7 gesenkt wurde. Nach Sterilfiltration wurde das resultierende Plasma in die Gefäße zur anschließenden Gefriertrocknung verfüllt. Die Rekonstitution der Lyophilisate erfolgte durch Zugabe von jeweils 184 ml Wasser für Injektionszwecke (WFI).
B. humanes Blutplasma / universell verwendbares Plasma wurde ohne Zugabe von pH-senkenden Lösungen hergestellt und verfüllt, Die Rekonstitution der Lyophilisate erfolgte durch Zugabe von jeweils 184 ml von WFI, das Zitronensäure in einer Konzentration von 3-4 mM enthielt.
C. humanes Blutplasma / universell verwendbares Plasma wurde wurde ohne Zugabe von pH-senkenden Lösungen hergestellt und verfüllt. Die Rekonstitution der Lyophilisate erfolgte durch Zugabe von jeweils 184 ml von WFI, jedoch ohne Zitronensäurezusatz,
D. humanes Blutplasma / universell verwendbares Plasma wurde in handelsübliche Beutel verfüllt und eingefroren gelagert. Das nichtlyophilisierte Produkt wurde als Kontrolle verwendet.

Repräsentative Gefäße jedes Ansatzes wurden rekonstituiert, wie oben beschrieben (bzw, nach Auftauen), und die individuellen pH-Werte gemessen. Zudem wurden wichtige Gerinnungsfaktoren (Faktoren II, V, VII, VIII, IX, X, XI, XII, XIII, ADAMTS13), Inhibitoren (Antithrombin III, Proteine C und S, Alpha-1-Antitrypsin, Antiplasmin und C1-Inhibitor) mit dem Fachmann bekannten Aktivitätstests quantifiziert. Zudem wurden sogenannte globale Gerinnungsparameter bestimmt, die einen Hinweis auf eventuelle Beeinträchtigungen eines oder mehrerer Faktoren geben können: aktivierte partielle Thromboplastinzeit (aPTT), Prothrombinzeit (PT), Thrombinzeit (TT) und Reptilasezeit (RT). Mögliche Aktivierungen von Faktoren zeigen sogenannte Marker an, wie der Thrombin-Antithrombin-Komplex (TAT), Prothrombinfragmente (F1+2), aktivierter Faktor VII (FVIIa) und D-Dimere.

### Ergebnis:

Individuelle pH-Werte
Ansatz A: pH 7,5 bis 7,9
Ansatz B: pH 7,3 bis 7,6
Ansatz C: pH 8,1 bis 8,4
Kontrolle: pH 7,0-7,4

Die Ansätze A und B wiesen für alle Testparameter, wie oben beschrieben, im Rahmen der Messgenauigkeiten keinen Verlust an Aktivitäten (+/-20% gemessen an den Kontrollen) auf. Eine signifikante Änderung der Konzentrationen an Aktivierungsmarkern wurde nicht festgestellt.

Dagegen zeigten die rekonstituierten Ansätze C gegenüber der Kontrolle erniedrigte Aktivitäten an Faktor VIII und Protein S, sowie eine verlängerte Reptilasezeit.

Um einen signifikanten Einfluss auf die Qualität des rekonstituierten Produktes zu vermeiden, kann die Einstellung des pH-Wertes vor Lyophilisierung erfolgen, um in den gewünschten pH-Bereich zu gelangen (A) oder durch Rekonstitution mit einer entsprechenden Lösung (B).

## Patentansprüche

1. Verfahren zur Rekonstitution eines Lyophilisats enthaltend Inhaltsstoffe, wobei
- die Rekonstitution eine Behandlung des Lyophilisats mit einer im wesentlichen wässrigen Lösung umfasst, die Substanzen enthält, die eine Einstellung eines vorbestimmten pH-Wert-Bereiches einer aus der Rekonstitution entstehenden Lösung bewirken, oder
- mit einer im wesentlichen wässrigen Lösung, wenn die Inhaltsstoffe bei dem Gefriertrocknungsprozess in einer Lösung vorlagen, die Substanzen enthielt, die die Einstellung eines vorbestimmten pH-Wert-Bereiches in der bei der Rekonstitution entstehenden Lösung bewirkten.

2. Verfahren nach Anspruch 1, wobei
- das Lyphilisat dadurch erhalten wird, dass die Inhaltsstoffe in einer im wesentlichen wässrigen Lösung vorliegen und in einen Behälter gefüllt werden, der zumindest teilweise aus für Wasser undurchlässigem aber für Wasserdampf durchlässigem und sterilem Material besteht und
- nach Beendigung des Gefriertrocknungsprozesses eine Aufnahme von Wasserdampf über einen vorher bestimmten Restfeuchtegehalt des Lyophilisats hinaus verhindert wird.

3. Verfahren nach Anspruch 2, wobei das für Wasser undurchlässige aber für Wasserdampf durchlässige und sterile Material auf einem Stützmaterial angeordnet ist.

4. Verfahren nach mindestens einem der vorhergehenden Ansprüche, wobei die Substanzen eine Einstellung des pH-Wertes in Bereiche, die die Struktur der Inhaltsstoffe wie Proteine im Lyophilisat durch zumindest teilweisen Verlust an Aktivität und/oder Nativität, Denaturierung, Fragmentierung, Aggregation, chemische Modifikation wie Oxidation negativ beeinflussen verhindern.

5. Verfahren nach mindestens einem der vorhergehenden Ansprüche, wobei die Substanzen Puffersubstanzen wie Salze aus mineralischen Säuren und Basen, insbesondere Phosphate oder Carbonate, insbesondere durch Einbringen von CO₂, Di- oder Monohydrogenphosphate, Hydrogencarbonate, Sulfate, Hydrogensulfate, Salze organischer Säuren oder Basen, Tris-Hydroxymethylaminomethan und/oder Aminosäuren sind

6. Verfahren nach mindestens einem der vorhergehenden Ansprüche, wobei die Inhaltsstoffe ausgewählt sind aus der Gruppe bestehend aus Biopolymeren wie Proteinen, Polysacchariden, Nukleinsäuren,Lipide und deren Mischungen.

7. Verfahren nach mindestens einem der vorhergehenden Ansprüche, wobei die Inhaltsstoffe Proteine aus Blut, Plasma oder Serum sind.

8. Verfahren nach mindestens einem der vorhergehenden Ansprüche, wobei Stabilisatoren wie Zucker, Polysaccharide, Oligosaccharide, Polyole, Aminosäuren oder Kombinationen davon im Lyophilisat oder in einer Rekonstitutionslösung vorhanden sind.

9. Verfahren nach mindestens einem der vorhergehenden Ansprüche, wobei die Biopolymeren als Inhaltsstoffe in biologischen Quellen wie Körperflüssigkeiten beispielsweise Blut oder Blutplasma, aus transgenen oder Fraktionen mit rekombinant hergestellten Proteinen enthalten sind.

10. Verfahren nach mindestens einem der Ansprüche 2 bis 9, wobei das für Wasserdampf durchlässige sterile Material aus fluorierten Kunststoffmaterialien, Polyester oder Polyethylen besteht.

11. Verfahren nach mindestens einem der Ansprüche 3 bis 10, wobei das Stützmaterial aus Polypropylen, Polyethylen, Ethylvinylacetat, Polyurethan, mehrschichtigen Polymeren wie Kraton® oder Kombinationen davon besteht.

12. Verfahren nach mindestens einem der vorhergehenden Ansprüche, wobei ein Gefriertrocknungsprozess angewendet wird, der einen ersten und einen zweiten Schritt umfasst, wobei im zweiten Schritt des Gefriertrocknungsprozess eine geringere Desorptionsenergieeintragung in den Gefriertrocknungsprozess als im ersten Schritt erfolgt.

13. Verfahren nach mindestens einem der Ansprüche 2 bis 12, wobei der für Wasserdampf durchlässige Behälter in einen für Wasserdampf undurchlässigen Behälter, der aus Kunststoffen oder Aluminiumfolie besteht, verbracht wird.

14. Verfahren nach mindestens einem der vorhergehenden Ansprüche, wobei die Rekonstitution des Lyophilisats in einer wässrigen Lösung erfolgt, die physiologisch verträgliche sauer hydrolysierende Salze oder physiologisch verträgliche Säuren enthält.

15. Verfahren nach mindestens einem der vorhergehenden Ansprüche, wobei die Rekonstitution mit im wesentlichen neutralem Wasser für Injektionszwecke erfolgt, wenn die Gefriertrocknung der Inhaltsstoffe in einer Lösung erfolgt, die Substanzen enthält, die die Einstellung eines vorbestimmten pH-Wert-Bereiches in der bei der Rekonstitution entstehenden Lösung bewirken.

16. Verwendung eines Behälters in einem Verfahren gemäß einem der Ansprüche 2 bis 15, wobei der Behälter zur Lyophilisierung Wände (10), Anschlusselemente (21, 22) aufweist und die Wände (10) zumindest teilweise aus für Wasser undurchlässigem aber für Wasserdampf durchlässigem und sterilem Material (15) besteht.
